# DEMANDE DE BREVET EUROPEEN

(11) **EP 0 779 671 A2**
(43) Date de publication de la demande: **18.06.1997**
(21) Numéro de dépôt: 96402698.3
(22) Date de dépôt: 11.12.1996
(51) Int. Cl.: H01M 6/16, C07C 311/48, C07C 303/36

(54) **Bis (phénylsulfonyl) imidures, leur procédé de préparation et matériaux à conduction ionique les comprenant**

(30) Priorité: 14.12.1995 FR 9514870
(71) Demandeur: ELECTRICITE DE FRANCE Service National, 75008 Paris (FR)
(72) Inventeur: Baudry, Paul, 77210 Avon (FR); Majestre, Hervé, 29000 Quimper (FR); Reibel, Léonard, 67800 Hoenheim (FR); Bayoud, Sami, 67000 Strasbourg (FR)
(74) Mandataire: Bernasconi, Jean

(57) **Abrégé**

L'invention concerne des matériaux à conduction ionique comprenant au moins un composé répondant à la formule (I) suivante : 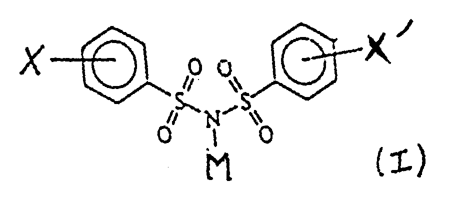 dans laquelle les groupes X et X' sont identiques ou différents et représentent au moins un groupement électroattracteur en position ortho, méta et/ou para et M représente un métal alcalin ou alcalino-terreux, en solution dans un solvant comprenant un matériau macromoléculaire ou un solvant ou mélange de solvant(s) aprotique(s) polaire(s). Les groupements électroattracteurs X,X' peuvent être choisis parmi les groupes nitro, perfluroroalkyles en C₁-C₅, CN, halogènes, vinyle, CO₂R et SO₂R où R représente un radical alkyle linéaire ou ramifié en C₁-C₅ et le métal M peut être choisi parmi le lithium, le sodium, le potassium, le césium, le magnésium, le calcium, le baryum.

L'invention concerne aussi un procédé de préparation des composés précités.

L'invention s'applique plus particulièrement à la fabrication de générateurs électrochimiques comprenant un électrolyte à base, au moins en partie, de ces matériaux à conduction ionique.

## Description

La présente invention se situe dans le domaine des matériaux à conduction ionique destinés à être utilisés notamment comme électrolytes dans les générateurs électrochimiques de courant.

Elle concerne plus particulièrement de nouveaux sels dénommés bis(phénylsulfonyl)imidures pouvant servir à la préparation de tels électrolytes.

L'invention concerne également un procédé de préparation de ces sels.

Elle concerne encore les matériaux à conduction ionique obtenus à partir de ces sels et les générateurs électrochimiques les contenant.

On connaît des sels ioniques susceptibles de conduire à la formation de solutions solides pouvant être utilisées dans la réalisation de générateurs électrochimiques de courant.

Le document EP-A-0 096 629 décrit notamment des perhalogénosulfonylimidures de métaux alcalins dans lesquels la chaîne perhalogénoalkyle comprend de 1 à 4 atomes de carbone, l'halogène étant de préférence le fluor et le métal alcalin étant de préférence le lithium ou le sodium.

Il décrit également la préparation d'électrolytes solides à partir de ces sels, par dissolution dans un matériau macromoléculaire tel que le poly(oxyde de propylène) et le poly(oxyde d'éthylène).

Les électrolytes solides ainsi obtenus peuvent servir à la réalisation de générateurs électrochimiques aussi bien primaires que secondaires.

On connaît également, pour la réalisation de tels générateurs, une autre classe d'électrolytes qui se présentent à l'état liquide.

Ainsi, le document FR-A-2 606 217 décrit un matériau à conduction ionique comprenant un sel en solution dans un solvant liquide aprotique polaire, le sel répondant à l'une des formules suivantes :

(I) M[RF-SO₂-N-SO₂-R'F]

(II) M[RF-SO₂-N-CO-R'F]

(III) M[RF-CO-N-CO-R'F]

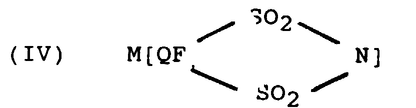

dans laquelle M est un métal alcalin, alcalino-terreux, un métal de transition ou un terre-rare ; RF et R'F sont identiques ou différents et représentent chacun un radical perhalogéné, de préférence perfluoré, ayant de 1 à 12 atomes de carbone ; QF est un radical divalent perfluoré ayant de 2 à 6 atomes de carbone.

Les éthers linéaires, les esters, les nitriles, les dérivés nitrés, les amides et les sulfones peuvent servir de solvant.

Ces électrolytes peuvent servir à la réalisation de générateurs rechargeables.

Récemment, il a également été décrit dans le document EP-A-0 482 287, un accumulateur électrochimique secondaire à électrolyte non-aqueux, dans lequel l'électrolyte non-aqueux résulte de la dissolution d'un sel dans un solvant contenant un ester cyclique et un ester linéaire. Le sel est de préférence choisi parmi le tétrafluoroborate de lithium, l'hexafluorophosphate de lithium, l'hexafluoroacétate de lithium, le trifluorométhanesulfonate de lithium et le perchlorate de lithium.

La présente invention a pour objectif de fournir de nouveaux composés pouvant servir à la préparation d'un matériau à conduction ionique, sous forme solide ou liquide, destinés notamment à la réalisation de générateurs électrochimiques de courant.

Un autre objectif est de fournir un nouveau procédé de préparation d'imidures symétriques ou dissymétriques.

La présente invention a encore pour objectif de fournir de nouveaux matériaux à conduction ionique utiles notamment comme électrolytes dans des générateurs électrochimiques.

A cette fin, l'invention a pour objet l'utilisation de composés répondant à la formule (I) suivante : 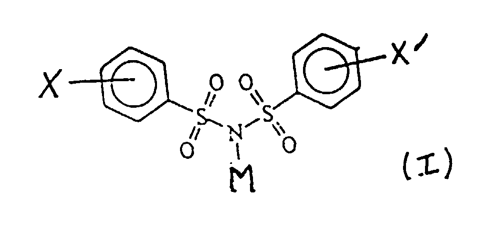 dans laquelle les groupes X et X' sont identiques ou différents et représentent au moins un groupement électro-attracteur en position ortho, méta et/ou para et M représente un métal alcalin ou alcalino-terreux, pour la préparation de matériau à conduction ionique.

L'invention a encore pour objet un procédé de préparation des composés de formule (I) ci-dessus, caractérisé en ce qu'il comprend les étapes consistant à :
a/ préparer le sulfonamide de formule (III) suivante : 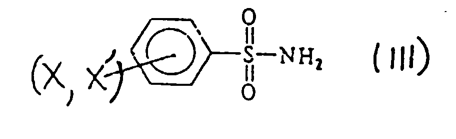 dans laquelle (X,X') représente au moins un groupement électro-attracteur en position ortho, méta et/ou para, à partir du chlorure de sulfonyle correspondant de formule (II) suivante : 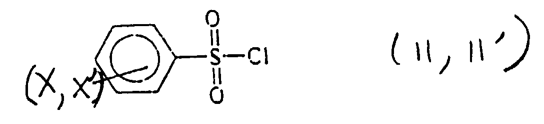 dans laquelle (X,X') est tel que défini précédemment, en présence d'ammoniaque aqueux ;
b/ préparer l'amidure de métal alcalin ou alcalino-terreux de formule (IV) suivante : 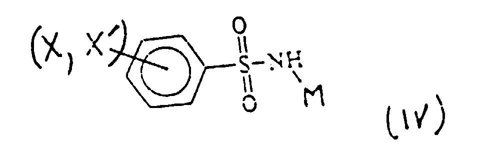 dans laquelle (X,X') et M ont les mêmes significations que précédemment, à partir du sulfonamide (III), à l'aide d'une base en milieu aqueux ;
c/ faire réagir l'amidure de formule (IV) avec le chlorure de sulfonyle de formule (II,II') en milieu aqueux pour former l'imidure (I) désiré.

L'invention a aussi pour objet un matériau à conduction ionique comprenant au moins un composé de formule (I) en solution dans un solvant solide ou liquide.

Enfin, l'invention a pour objet un générateur électrochimique, caractérisé en ce qu'il comprend un électrolyte formé, au moins en partie, d'un matériau à conduction ionique tel que précité.

Les composés mis en oeuvre selon l'invention répondent à la formule (I) donnée ci-dessus.

Selon l'invention, le terme "phényle" désigne un radical phényle au moins mono-substitué par un groupement électroattracteur X ou X'.

Ces groupements électroattracteurs sont avantageusement choisis parmi les groupes nitro, perfluoroalkyle en C₁-C₅, CN, halogènes (de préférence F ou Br), vinyle CO₂R,'et SO₂R où R représente un radical alkyle linéaire ou ramifié en C₁-C₅. Les groupes nitro et trifluorométhyle sont préférés.

Le ou les groupements électroattracteurs X ou X' peuvent se situer en position ortho, méta et/ou para.

Le radical phényle peut être mono-substitué en position ortho, méta ou para, la position para étant préférée.

Selon l'invention, M représente un métal choisi notamment parmi le groupe comprenant Li, Na, K, Cs, Mg, Ca, Ba. Il représente de préférence le lithium ou le sodium, le lithium étant le métal le plus préféré.

En ce qui concerne l'obtention d'imidures symétriques, on trouve dans la littérature (G. DAUPHIN et A. KERGOMARD, Bull. Soc. Chim. Fr., 3, 486 (1961); US-A-2 348 226) des exemples de préparation par couplage direct de deux équivalents du chlorure de sulfonyle correspondant en présence de NH₄OH et en milieu aqueux en maintenant le pH à 10-11 par addition de NaOH.

Néanmoins, lorsque l'on souhaite préparer des imidures de lithium et non plus de sodium en remplaçant la soude par la lithine, cette méthode conduit à des mélanges de produits et n'est donc pas satisfaisante.

Les inventeurs ont trouvé une nouvelle méthode de préparation d'imidures permettant d'obtenir le produit désiré avec de bons rendements.

Ils ont montré que l'on pouvait obtenir l'imidure par couplage de l'amidure (IV) correspondant, préparé à partir du chlorure de sulfonyle (II), avec le chlorure de sulfonyle de départ (II) dans le cas d'imidures symétriques ou avec un autre chlorure de sulfonyle (II') convenablement substitué dans le cas d'imidures dissymétriques (X différent de X').

D'une manière plus précise, on utilise comme matière de départ un chlorure de sulfonyle de formule (II) suivante : 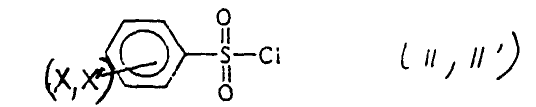 portant un groupement phényle convenablement substitué par au moins un groupement électro-attracteur (X,X') (c'est-à-dire un groupement phényle correspondant au groupement phényle substitué du produit final désiré) que l'on traite avec de l'ammoniaque aqueux en excès, ce qui conduit au sulfonamide correspondant de formule (III) suivante :

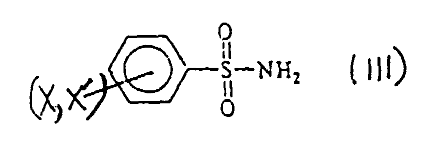

Le sulfonamide (III) est isolé par filtration après précipitation en acidifiant le milieu.

On transforme ensuite ce sulfonamide (III) en amidure correspondant de formule (IV) suivante : 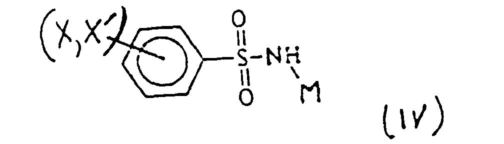 par traitement à l'aide d'une solution aqueuse basique, par exemple une solution de LiOH ou NaOH selon que l'on souhaite obtenir l'amidure de lithium ou de sodium.

On obtient finalement l'imidure (I) désiré par couplage en milieu aqueux de l'amidure (IV) précédemment obtenu avec du chlorure de sulfonyle (II) utilisé également comme produit de départ. L'imidure ainsi obtenu est symétrique (X et X' sont identiques).

Conformément à l'invention, on utilise avantageusement deux équivalents de sulfonamide (IV) préparé au préalable, que l'on fait réagir directement avec un équivalent de chlorure de sulfonyle (II) dans l'eau.

Les inventeurs ont trouvé qu'il était plus efficace d'utiliser l'amidure (IV) à la fois comme nucléophile et comme base plutôt que d'utiliser de la lithine ou de la soude comme base.

En outre, parallèlement à l'imidure (I), on recueille également le sulfonamide (III) qui peut ainsi être recyclé pour l'obtention de l'amidure correspondant (IV).

Selon l'invention, on peut également préparer des imidures dissymétriques c'est-à-dire des composés de formule (I) dans laquelle X et X' sont différents.

Dans ce cas, on procède au couplage de l'amidure (IV) portant le ou les substituants X désiré(s) avec du chlorure de sulfonyle (II') portant le ou les substituants X' désiré(s), ou inversement.

Les composés (I) selon l'invention sont particulièrement adaptés pour la préparation de matériaux à conduction ionique.

En effet, conformément à l'invention, on peut obtenir de tels matériaux par dissolution d'au moins un composé (I) dans un solvant solide ou liquide.

Le solvant solide peut être constitué d'un matériau macromoléculaire formé, au moins en partie, par un polymère dont les motifs monomères comportent au moins un hétéroatome, notamment l'oxygène ou l'azote, apte à former des liaisons de type donneur-accepteur avec le ou les composé(s) de formule (I).

Le rapport du nombre d'hétéroatomes provenant des motifs monomères dudit polymère au nombre d'atomes de métal alcalin, est avantageusement compris entre 4 et 100 et de préférence entre 8 et 30.

Les matériaux macromoléculaires utilisés selon l'invention peuvent consister en des homopolymères ou des copolymères

Il s'agit de préférence du poly(oxyde d'éthylène) ou du poly(oxyde de propylène).

La proportion du ou des composés (I) est choisie, bien entendu, de façon à être inférieure au seuil de solubilité de ce(s) composé(s) dans le matériau macromoléculaire selectionné.

La préparation du matériau à conduction ionique est réalisée par toute méthode connue.

Elle peut ainsi s'effectuer par mise en solution dans un solvant à la fois pour le polymère et le(s) composé(s) (I), tel que par exemple l'acétonitrile ou le méthanol, puis élimination de ce solvant.

On peut encore procéder sans solvant par dissolution du ou des composés (I) dans le polymère à l'état fondu.

Selon l'invention, on peut également préparer un matériau à conduction ionique par dissolution d'au moins un composé (I) dans un solvant liquide.

Il peut s'agir d'un solvant aprotique polaire choisi parmi le groupe comprenant :
- les éthers linéaires tels que le diéthyléther, le diméthoxyéthane ou les éthers cycliques tels que le tétrahydrofuranne, le dioxanne, le diméthyltétrahydrofuranne ;
- les esters tels que le formiate de méthyle ou d'éthyle, le carbonate de propylène ou d'éthylène, les butyrolactones ;
- les nitriles tels que l'acétonitrile et les benzonitriles ;
- les dérivés nitrés tels que le nitrométhane ou le nitrobenzène ;
- les amides tels que le diméthylformamide, le diéthylformamide et la N-méthylpyrrolidone ;
- les sulfones tels que la diméthylsulfone, tétraméthylsulfone, les lactones et α-valerolactones et le sulfolane.

On peut aussi utiliser un mélange de ces solvants.

Comme dans le cas des matériaux solides, la proportion du ou des composé(s) (I) doit rester inférieure au seuil de solubilité de ce(s) composé(s) dans le solvant ou mélange de solvants utilisé.

Les matériaux à conduction ionique ainsi obtenus présentent une conductivité permettant de les utiliser en particulier comme électrolytes pour générateurs électrochimiques de courant.

Selon l'invention, on peut ainsi préparer des électrolytes solides ou liquides pour générateurs électrochimiques primaires ou secondaires pouvant être également de type rechargeable.

Pour la réalisation d'un générateur électrochimique, on associe un électrolyte formé, au moins en partie, d'un matériau à conduction ionique telle que décrit précédemment, à une électrode négative en un matériau apte à fournir des ions alcalins correspondant au métal alcalin M du composé (I) choisi et à une électrode positive en un matériau apte à incorporer les atomes de ce métal.

On peut, par exemple, employer une électrode négative formée d'un alliage, d'un composé intermétallique ou d'un composé d'insertion à base de ce métal.

L'électrode positive peut, quant à elle, être formée de tout matériau à structure cristalline permettant l'insertion de métaux alcalins.

La présence de composés (I) selon l'invention dans des électrolytes pour générateur en particulier du type accumulateur rechargeable permet avantageusement de retarder l'apparition d'arborescences de type dendritique lors de la recharge de l'accumulateur et a ainsi pour effet d'améliorer la cyclabilité de celui-ci.

On ajuste avantageusement la concentration en composé(s) (I) pour obtenir à la fois une conductivité ionique et un effet anti-dendrites satisfaisants.

Selon l'invention, on peut prévoir également un électrolyte comprenant au moins un composé (I) en combinaison avec un autre sel présentant des propriétés de conduction tel qu'un bis(perhalogénoalkylsulfonyl) imidure de métal alcalin ou alcalino terreux, notamment le bis(trifluorométhylsulfonyl) imidure de lithium.

Pour une électrolyte à base de poly(oxyde d'éthylène) POE par exemple, contenant au moins un composé (I) et un autre sel présentant des propriétés de conduction du type précité, la concentration en composé(s) (I) par rapport à la concentration totale des sels (imidures) présents dans le POE est de préférence comprise entre 1 et 70 % molaire, plus préférentiellement encore entre 1 et 30 % molaire.

D'autres caractéristiques et avantages de l'invention ressortiront des exemples donnés ci-après à titre illustratif et non limitatif ainsi que des dessins annexés dans lesquels :
- les figures 1A et 1B représentent les graphes d'analyse thermogravimétrique entre 35°C et 798°C en atmosphères inerte et oxydante, pour un imidure de formule (I) selon l'invention dans laquelle X = X' et représente un groupe nitro en position para et M représente le lithium.
- la figure 2 représente les courbes de conductivité en fonction de la température pour un matériau à conduction ionique selon l'invention comprenant un imidure de formule (I) dans laquelle X = X' et représente un groupe nitro en position para et M représente le lithium, en solution dans le poly(oxyde d'éthylène), à différentes concentrations de sel ;
- la figure 3 montre une courbe indiquant la durée à l'issue de laquelle se produit la mise en court-circuit pour un accumulateur rechargeable muni d'un électrolyte polymère comprenant un composé (I) selon l'invention ;
- la figure 4 montre une courbe indiquant la durée à l'issue de laquelle se produit la mise en court-circuit pour un accumulateur rechargeable muni d'un électrolyte polymère connu.

### EXEMPLES

Dans tout ce qui suit, le symbole "X-Ph" ou "X'-Ph" représente un radical phényle possèdant au moins un substituant électroattracteur X ou X' et "p" signifie la position para.

Les déplacements chimiques des spectres RMN sont exprimés en ppm et il est indiqué entre parenthèses la multiplicité (s = singulet, d = doublet), le nombre d'hydrogènes et l'attribution (ArH = H aromatique).

### Exemple 1 : PREPARATION DU BIS(PARA-NITROPHENYLSULFONYL) IMIDURE DE LITHIUM

### Etape a : préparation du para-nitrophénylsulfonamide (III avec X = p-NO₂)

On prépare le sulfonamide (III) dans lequel le groupement phényle est substitué en para par un groupe nitro (p-NO₂-Ph-SO₂NH₂), en traitant le chlorure de sulfonyle (II) correspondant (p-NO₂-Ph-SO₂Cl) par une solution aqueuse ammoniacale en excès selon la méthode décrite dans la littérature (G.DAUPHIN et A. KERGOMARD, Bull. Soc. Chim. Fr., 3, 486 (1961)).

Après réaction, on acidifie le milieu avec de l'acide chlorhydrique et on filtre pour récupérer le sulfonamide p-NO₂-Ph-SO₂NH₂ précipité.

On purifie le produit obtenu par lavage à l'eau et au dichlorométhane. 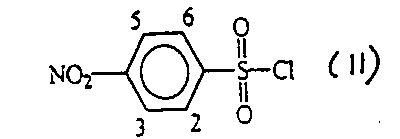
- para-nitrophénylsulfochlorure (II) :
   RMN-¹H (DMSO-d₆) : 8,21, 8,17 (d, 2H, ArH5 et H3) ; 7,84, 7,81 (d, 2H, ArH6 et H2).
- paranitrophénylsulfonamide (III) :
   RMN-¹H (DMSO-d₆) : 8,43, 8,38 (d, 2H, ArH5 et H3) ; 8,08, 8,03 (d, 2H, ArH6 et H2) ; 7,72 (s, 2H, NH₂).

### Etape b : préparation du para-nitrophénylsulfonylamidure de lithium (IV avec X = p-NO₂ et M = Li)

On traite le sulfonamide (III) par une solution aqueuse comprenant un équivalent de lithine. Après réaction le produit est isolé par évaporation du solvant.

RMN-¹H (DMSO-d₆) : 8,17, 8,14 (d, 2H, ArH5 et H3) ; 7,88, 7,84 (d, 2H, ArH6 et H2) ; 3,3 (pic large, 1H, -NH-).

### Etape c : préparation du bis (para-nitrophénylsulfonyl)imidure de lithium (I avec X = NO₂ et M = Li)

On fait réagir deux équivalents de sulfonamidure (IV) obtenu précédemment à l'étape b/ avec un équivalent du sulfochlorure (II) de départ, en présence d'eau.

Parallèlement à la formation de l'imidure (I) recherché, il se forme également du para-nitrosulfonamide (III) qui précipite hors de la solution aqueuse et qui est réutilisable par la suite. L'imidure (I) est isolé par évaporation du solvant, puis recristallisé après redissolution à chaud dans l'eau.

Après recristallisation de l'imidure (I) obtenu par concentration de la solution aqueuse et resolubilisation à chaud, on le fait sécher sous vide à 80°C pendant 24 heures, ce qui conduit au produit pur avec un rendement de 88 %.
- L'analyse élémentaire du produit ainsi isolé confirme l'absence de tout autre produit.
- RMN-¹H (DMSO-d₆, D₂O) : 8,24, 8,21 (d, 4H, ArH3, H5, H3', H5') ; 7,91, 7,87 (d, 4H, ArH2, H6, H2', H6'). 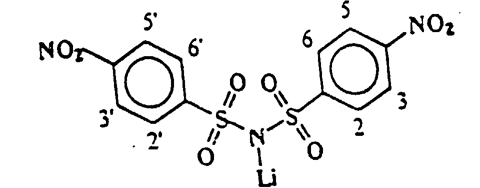
- RMN⁻¹³C (DMSO-d₆, D₂O) : 151,6 (C4 et C4') ; 145,4 (C1 et C1') ; 126,4 (C2 et C2', C6 et C6') ; 123,9 (C3 et C3', C5 et C5').

L'affectation des différentes raies a été réalisée à l'aide du spectre de l'arylsulfonamidure de lithium et en utilisant le logiciel CHEMWIND pour prévoir les déplacements des carbones de l'imidure.
- Analyse thermogravimétrique : Elle est réalisée entre 35°C et 798°C avec une vitesse de montée en température de 10°/minute en atmosphères inerte et oxydante.

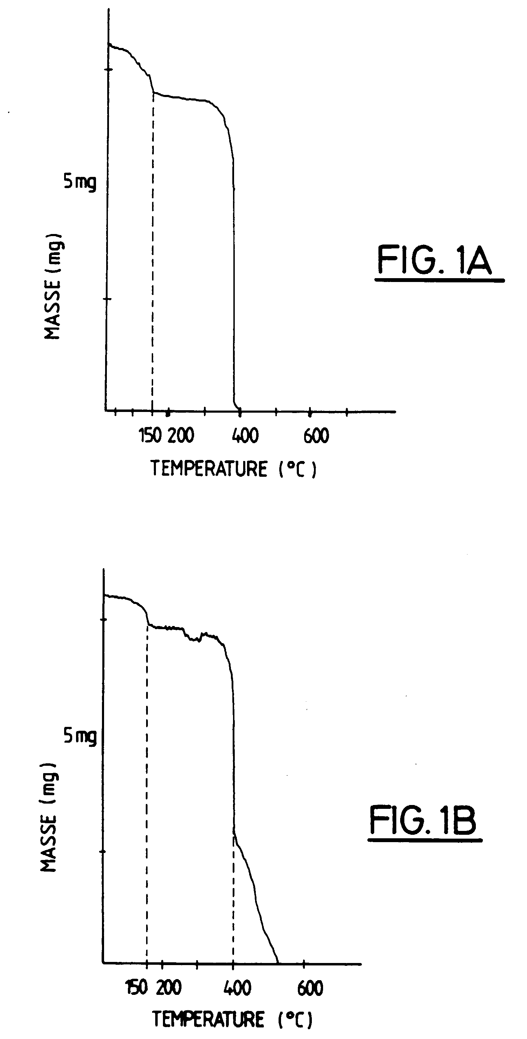

Les graphes obtenus sont donnés aux figures 1A (dans N₂) et 1B (dans l'air).

Ces graphes indiquent la présence d'eau qui est éliminée à 150°C. Cette valeur élevée indique une forte énergie de solvatation du sel par l'eau.

Ils montrent également que le sel (I) est stable jusqu'à 370°C en milieu inerte ou oxydant (air ambiant).

### EXEMPLE 2 : PREPARATION DU 4-NITRO-4'-VINYL BIPHENYL SULFONYLIMIDURE DE LITHIUM.

On procède comme à l'exemple 1 pour la préparation du parastyrènesulfonamidure de lithium (IV avec X'=p-vinyle et M=Li) à partir du parastyrènesulfochlorure (II' avec X'=p-vinyle).
- parastyrène sulfochlorure :
   RMN-¹H (DMSO-d₆) : 7,58, 7,54 (d, 2H, ArH2' et H6') 7,43, 7,39 (d, 2H, ArH3' et H5') ; 6,79, 6,73, 6,70, 6,65 (dd, 1H, =CH-) ; 5,87, 5,78 (d, 1H, H2C=) ; 5,29, 5,24 (d, 1H, H2C=).
- parastyrène sulfonamidure :
   RMN-¹ (DMSO-d6) : 7,58, 7,54 (d, 2H, ArH2' et H6') ; 7,43, 7,39 (d, 2H, ArH3' et H5') ; 6,79, 6,74, 6,70, 6,65 (dd, 1H, =CH-) ; 5,87, 5,78 (d, 1H, H2C=), 5,29, 5,23 (d, 1H, H2C=).

On procède ensuite au couplage du parastyrène sulfonamidure (IV) ainsi préparé avec le para-nitrophénylsulfochlorure (II) dans les conditions énoncées à l'étape c) de l'exemple 1. On obtient ainsi l'imidure dissymétique désiré 4-nitro-4'-vinylbiphényl sulfonylimidure de lithium.
- RMN-¹H (DMSO-d₆) : 8,21, 8,17 (d, 2H, ArH5 et H3) ; 7,87, 7,83 (d, 2H, ArH6 et H2) ; 7,60, 7,56 (d, 2H, ArH2' et H6') ; 7,46, 7,42 (d, 2H, ArH3 ' et H5') ; 6,81, 6,75, 6,72, 6,66 (dd, 1H =CH-) ; 5,92, 5,83 (d, 1H, H2C=) ; 5,36, 5,30 (d, 1H, H2C=).
- RMN-¹³C : 151,6 (C4) ; 145,4 (C1) ; 140,6 (C4') ; 138,5 (Cl') ; 135,8 (CH vinyle) ; 126,5 (C3'et C5') ; 126,4 (C2 et C6) ; 125,4 (C2' et C6') ; 123,9 (C3 et C5) ; 112,3 (CH₂ vinyle).

L'affectation des différentes raies a été réalisée à l'aide du spectre de l'Aryle sulfonamidure de lithium et en utilisant le logiciel Chemwind pour prévoir les déplacements des carbones de l'imidure.
- analyse élémentaire :
   . après évaporation sous vide à 80°C
      % C = 43,61 ; % H = 3,11 ; % N = 7,34
      % Li = 1,770 ; % H₂O = 2,934.
   . après traitement à l'acétonitrile et évaporation sous vide à 50°C
      % C = 43,99 ; % H = 3, 62 ; % N = 6,58 % ;
      % Li = 1,545 %, % H₂O = 2,658 %.

### ETUDE DE LA CONDUCTIVITE

Le sel obtenu dans l'exemple 1 donné ci-dessus a été étudié pour ses propriétés de conduction dans un système d'électrolyte polymère.

On a ainsi préparé plusieurs échantillons de poly(oxyde d'éthylène) POE 900.10³ contenant du bis (para-nitrophénylsulfonyl)imidure de lithium (NO₂PhSILi) avec des teneurs en sel différentes représentées par le rapport O/Li.

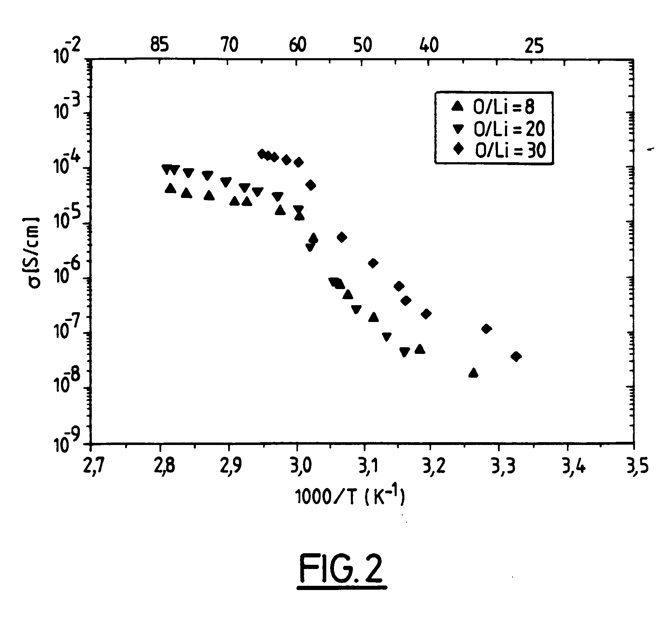

La figure 2 donne les courbes de conductivité obtenues en fonction de 1/T, les mesures étant faites à températures croissantes.

On peut constater que les meilleurs résultats sont obtenus avec un rapport O/Li = 30.

Les conductivités sont faibles à température ambiante (10⁻⁸S/cm) du fait de la cristallinité du POE, puis augmentent rapidement pour atteindre les 10⁻⁴ S/cm à 60°C dans le cas de O/Li = 30.

### EXEMPLE 3 : FABRICATION D'UN ACCUMULATEUR RECHARGEABLE.

On forme une cellule électrochimique de 7cm² constituée de deux électrodes en lithium séparées par un électrolyte polymère à base de poly(oxyde d'éthylène) POE et comprenant un mélange de bis(trifluorométhylsulfonyl) imidure de lithium(LiTFSI) et de bis(para-nitrophénylsulfonyl) imidure de lithium (LiNPSI) selon un rapport molaire LiNPSI/LiTFSI égal à 0,5 de tel sorte que le rapport O/li global (tenant compte des deux sels de lithium présents) soit de 30.

### ETUDE DE L'EFFET ANTI-DENDRITES

A partir d'une cellule électrochimique selon l'exemple 3, on mesure le temps à l'issu duquel la cellule est mise en court-circuit sous l'effet de la croissance dendritique, pour un courant constant appliqué de 0,1 mA/cm².

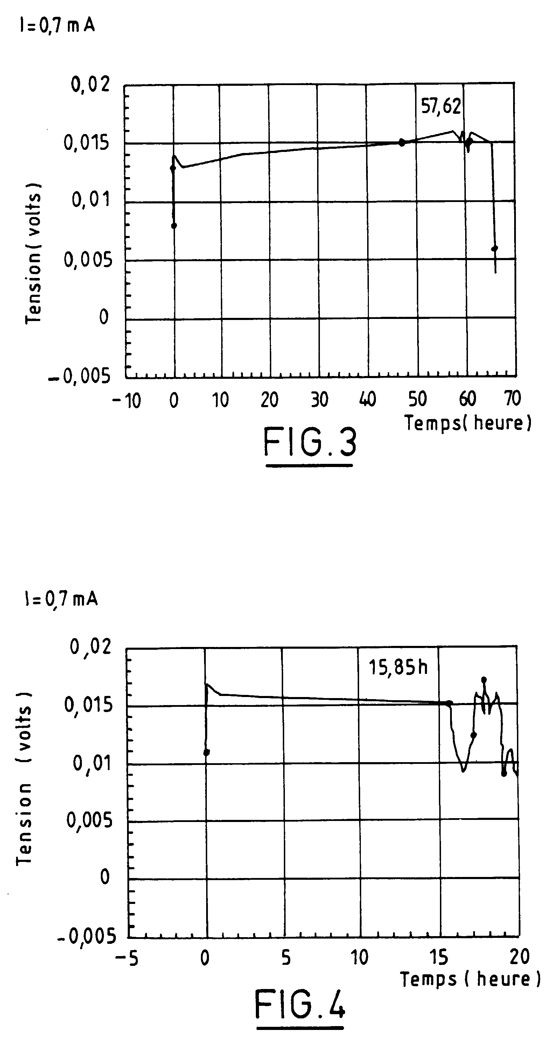

La figure 3 montre les résultats obtenus.

A titre comparatif, on réalise une cellule électrochimique du même type qu'à l'exemple 3, à l'exception de l'électrolyte à base de POE, qui comprend uniquement du bis(trifluorométhylsulfonyl) imidure de lithium (LiTFSI) selon un rapport O/Li = 30.

La figure 4 montre les résultats obtenus pour cet exemple comparatif.

On voit que lorsque le composé selon l'invention est présent (LiNPSI), le court-circuit ne se produit qu'après environ 60 heures alors que dans le cas de l'électrolyte connu, le court-circuit se produit après seulement environ 15 heures.

Ceci indique que la présence d'un composé selon l'invention permet d'obtenir un dépôt de lithium plus homogène comparativement au cas où il est absent.

## Revendications

1. Matériau à conduction ionique, caractérisé en ce qu'il comprend au moins un composé de formule (I) suivante : 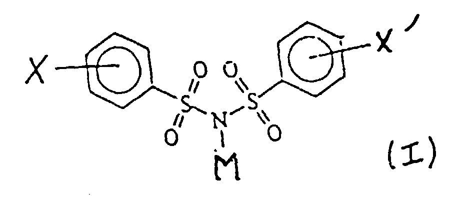 dans laquelle les groupes X et X' sont identiques ou différents et représentent au moins un groupement électro-attracteur en position ortho, méta et/ou para et M représente un métal alcalin ou alcalino terreux,
en solution dans un solvant contenant un matériau macromoléculaire ou un solvant ou un mélange de solvant(s) aprotique(s) polaire(s).

2. Matériau à conduction ionique selon la revendication 1, caractérisé en ce que les groupements électroattracteurs X,X' sont choisis parmi les groupes nitro, perfluroroalkyles en C₁-C₅, CN, halogènes, vinyle, CO₂R et SO₂R où R représente un radical alkyle linéaire ou ramifié en C₁-C₅.

3. Matériau à conduction ionique selon l'une quelconque des revendications 1 et 2, caractérisé en ce que les groupements électroattracteurs sont choisis parmi les groupes nitro et trifluorométhyle.

4. Matériau à conduction ionique selon l'une quelconque des revendications 1 à 3, caractérisé en ce que le métal M est choisi parmi le groupe comprenant le lithium, le sodium, le potassium, le césium, le magnésium, le calcium, le baryum.

5. Matériau à conduction ionique selon l'une quelconque des revendications 1 à 4, caractérisé en ce que le métal M est un métal alcalin choisi parmi le lithium ou le sodium.

6. Matériau à conduction ionique selon l'une quelconque des revendications 1 à 5, caractérisé en ce que le radical phényle est mono-substitué en position para.

7. Matériau à conduction ionique selon l'une quelconque des revendications 1 à 6, caractérisé en ce qu'il comprend au moins un composé choisi parmi le bis(para-nitrophénylsulfonyl) imidure de lithium et le (4-nitro-4'-vinyl-diphénylsulfonyl) imidure de lithium.

8. Matériau à conduction ionique selon l'une quelconque des revendications 1 à 7, caractérisé en ce que le solvant est un solvant solide constitué d'un matériau macromoléculaire formé, au moins en partie, par un polymère dont les motifs monomères comportent au moins un hétéroatome, notamment d'oxygène ou d'azote, apte à former des liaisons du type donneur-accepteur avec le composé (I).

9. Matériau à conduction ionique selon la revendication 8, caractérisé en ce que le rapport du nombre d'hétéroatomes provenant des motifs monomères dudit matériau macromoléculaire au nombre d'atome de métal alcalin du composé (I) est compris entre 4 et 100, de préférence entre 8 et 30.

10. Matériau à conduction ionique selon l'une quelconque des revendications 1 à 9, caractérisé en ce que le matériau macromoléculaire comprend un polymère choisi parmi le poly(oxyde d'éthylène) et le poly(oxyde de propylène).

11. Matériau à conduction ionique selon l'une quelconque des revendications 1 à 7, caractérisé en ce que le solvant est un solvant liquide formé d'un solvant ou d'un mélange de solvants aprotique(s) polaire(s) choisi(s) parmi le groupe comprenant :
- les éthers linéaires tels que le diéthyléther, le diméthoxyéthane ou les éthers cycliques tels que le tétrahydrofuranne, le dioxanne, le diméthyltétrahydrofuranne ;
- les esters tels que le formiate de méthyle ou d'éthyle, le carbonate de propylène ou d'éthylène, les butyrolactones ;
- les nitriles tels que l'acétonitrile et les benzonitriles ;
- les dérivés nitrés tels que le nitrométhane ou le nitrobenzène ;
- les amides tels que le diméthylformamide, le diéthylformamide et la N-méthylpyrrolidone ;
- les sulfones tels que la diméthylsulfone, tétraméthylsulfone, les lactones et α-valérolactones et le sulfolane.

12. Matériau à conduction ionique selon l'une quelconque des revendications précédentes, caractérisé en ce qu'il comprend en outre au moins un autre sel présentant des propriétés de conduction tel qu'un bis(perhalogénoalkylsulfonyl) imidure de métal alcalin ou alcalino-terreux, notamment le bis(trifluorométhylsulfonyl) imidure de lithium.

13. Matériau à conduction ionique selon la revendication 12, caractérisé en ce que la concentration en composé(s) (I) par rapport à la concentration totale des sels conducteurs est comprise entre 1 et 70 % molaire, de préférence 1 à 30 % molaire.

14. Utilisation d'un composé de formule (I) suivante : 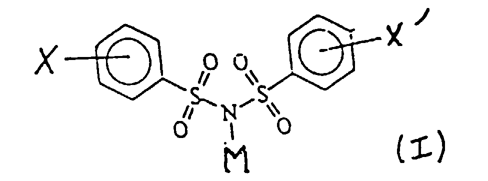 dans laquelle les groupes X et X' sont identiques ou différents et représentent au moins un groupement électro-attracteur en position ortho, méta et/ou para et M représente un métal alcalin ou alcalino terreux, pour la préparation d'un matériau à conduction ionique.

15. Utilisation selon la revendication 14, caractérisée en ce que les groupements électroattracteurs X,X' sont choisis parmi les groupes nitro, perfluroroalkyles en C₁-C₅, CN, halogènes, vinyle, CO₂R et SO₂R où R représente un radical alkyle linéaire ou ramifié en C₁-C₅.

16. Utilisation selon l'une quelconque des revendications 14 ou 15, caractérisée en ce que les groupements électroattracteurs sont choisis parmi les groupes nitro et trifluorométhyle.

17. Utilisation selon l'une quelconque des revendications 14 à 16, caractérisée en ce que le métal M est choisi parmi le groupe comprenant le lithium, le sodium, le potassium, le césium, le magnésium, le calcium, le baryum.

18. Utilisation selon l'une quelconque des revendications 14 à 17, caractérisée en ce que le métal M est un métal alcalin choisi parmi le lithium ou le sodium.

19. Utilisation selon l'une quelconque des revendications 14 à 18, caractérisée en ce que le radical phényle est mono-substitué en position para.

20. Utilisation selon l'une quelconque des revendications 14 à 19, caractérisée en ce que le composé de formule (I) est choisi parmi le bis(para-nitrophénylsulfonyl) imidure de lithium et le (4-nitro-4'-vinyl-diphénylsulfonyl) imidure de lithium.

21. Utilisation selon l'une quelconque des revendications 14 à 20, pour retarder la formation de dendrites.

22. Utilisation selon l'une quelconque des revendications 14 à 21, caractérisée en ce que le matériau à conduction ionique comprend en outre au moins un autre sel présentant des propriétés de conduction tel qu'un bis(perhalogénoalkylsulfonyl)imidure de métal alcalin ou alcalino-terreux, notamment le bis(trifluorométhylsulfonyl)imidure de lithium.

23. Utilisation selon la revendication 22, caractérisée en ce que la concentration en composé(s) (I) par rapport à la concentration totale des sels conducteurs est comprise entre 1 et 70 % molaire, de préférence 1 à 30 % molaire.

24. Générateur électrochimique, caractérisé en ce qu'il comprend un électrolyte formé, au moins en partie, d'un matériau à conduction ionique selon l'une quelconque des revendications 1 à 13, une électrode négative en un matériau apte à fournir des ions de métal alcalin ou alcalino-terreux M correspondant au métal M du composé de formule (I) présent dans ledit matériau à conduction ionique et une électrode positive en un matériau apte à incorporer les ions M.

25. Générateur électrochimique selon la revendication 24, caractérisé en ce qu'il consiste en un accumulateur rechargeable et en ce qu'il comprend une quantité efficace pour retarder la formation de dendrites, dudit matériau à conduction ionique.

26. Procédé pour générer un courant, caractérisé en ce que l'on utilise un matériau à conduction ionique selon l'une quelconque des revendications 1 à 13, une électrode négative en un matériau apte à fournir des ions de métal alcalin ou alcalino-terreux M correspondant au métal M du composé de formule (I) présent dans ledit matériau à conduction ionique et une électrode positive en un matériau apte à incorporer les ions M.

27. Procédé pour générer un courant selon la revendication 26, caractérisé en ce qu'il est mis en oeuvre dans un accumulateur rechargeable.

28. Procédé de préparation de composés de formule (I) suivante : 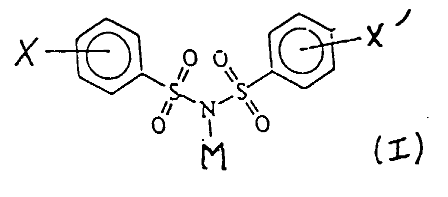 dans laquelle les groupes X et X' sont identiques ou différents et représentent au moins un groupement électro-attracteur en position ortho, méta et/ou para et M représente un métal alcalin ou alcalino terreux, caractérisé en ce qu'il comprend les étapes consistant à :
a/ préparer le sulfonamide de formule (III) suivante : 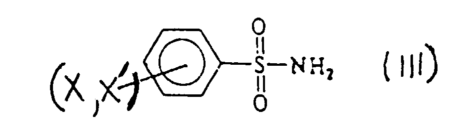 dans laquelle (X, X') représente au moins un groupement électroattracteur en position ortho, méta et/ou para, à partir du chlorure de sulfonyle correspondant de formule (II,Il') suivante : 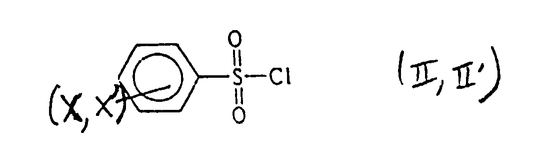 dans laquelle (X,X') est tel que défini précédemment, en présence d'ammoniaque aqueux ;
b/ préparer l'amidure de métal alcalin ou alcalino-terreux de formule (IV) suivante : 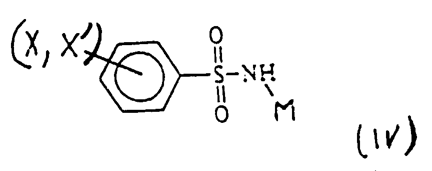 dans laquelle (X,X') et M ont la même signification que précédemment, à partir du sulfonamide (III), à l'aide d'une base en milieu aqueux ;
c/ faire réagir l'amidure de formule (IV) avec le chlorure de sulfonyle de formule (II,II') en milieu aqueux pour former l'imidure (I) désiré.

29. Procédé selon la revendication 28, caractérisé en ce que, à l'étape c/, on fait réagir deux équivalents d'amidure (IV) avec un équivalent de chlorure de sulfonyle (II,II').

30. Procédé selon l'une quelconque des revendications 28 ou 29, caractérisé en ce que les groupements électroattracteurs X,X' sont choisis parmi les groupes nitro, perfluroroalkyles en C₁-C₅, CN, halogènes, vinyle, CO₂R et SO₂R où R représente un radical alkyle linéaire ou ramifié en C₁-C₅.

31. Procédé selon l'une quelconque des revendications 28 à 30, caractérisé en ce que le métal M est choisi parmi le groupe comprenant le lithium, le sodium, le potassium, le césium, le magnésium, le calcium, le baryum.

32. Procédé selon l'une quelconque des revendications 28 à 31, caractérisé en ce que l'un de X ou X' représente un groupe nitro en position para et l'autre de X et X' représente un groupe nitro ou un groupe vinyle, en position para, M étant le lithium.
